# EUROPEAN PATENT SPECIFICATION

(11) **EP 1 253 861 B1**
(45) Date of publication and mention of the grant of the patent: **06.09.2006**
(21) Application number: 01960507.0
(22) Date of filing: 17.07.2001
(51) Int. Cl.: A61B 17/115

(54) **SURGICAL STAPLING INSTRUMENT**
CHIRURGISCHES KLAMMERINSTRUMENT
INSTRUMENT CHIRURGICAL D'AGRAFAGE

(30) Priority: 01.08.2000 DE 10037453
(43) Date of publication of application: 06.11.2002
(73) Proprietor: Ethicon Endo-Surgery (Europe) GmbH, 22851 Norderstedt (DE)
(72) Inventor: LONGO, Antonio, I-90134 Palermo (IT); CORSARO, Santi, Irvine, CA 92618 (US)
(74) Representative: UEXKÜLL & STOLBERG
(86) International application number: PCT/EP2001/008242
(87) International publication number: WO 2002/009595

(56) References cited:
- US-A- 4 752 024
- US-A- 5 205 459
- US-A- 5 957 363

## Description

The invention relates to a surgical stapling instrument, which can be used for applying surgical staples or clips to tissue and in particular for performing an anastomosis.

Generally, in the performance of a surgical anastomotic stapling operation, two pieces of lumen or tubular tissue, e.g., intestinal tissue, are attached together by a closed row of staples. In performing the anastomosis with a surgical stapling instrument, the two pieces of tubular tissue are clamped together between an anvil provided with an array of staple forming grooves and a staple holder or cartridge device provided with a plurality of staple receiving slots arranged in a closed row or array in which the staples are received. A staple pusher is advanced to drive the staples into the tissue and form the staples against the anvil. Moreover, a circular knife is advanced to cut the excess tissue clamped between the anvil and the staple holder. As a result, the donut-shaped section of tissue is severed from each lumen and remains on the anvil shaft. The tubular tissue joined by the closed row of staples is unclamped by moving the anvil relative to the staple holder, usually by advancing the anvil shaft distally to move the anvil away from the staple holder. The stapling instrument is removed by pulling the anvil through the opening between the pieces of tubular tissue attached by the array of staples.

Surgical stapling instruments of this kind are well-known. For example, U.S. patent No. 5 205 459 describes such an instrument in detail. As usual, the closed row of staples of the instrument disclosed has a circular shape.

Although the use of the known surgical stapling instruments is very beneficial and greatly facilitates the performance of an anastomosis, it involves some problems. Often it is difficult to retract the instrument from the site of the operation, because it is difficult to move the anvil through the opening bordered by the closed row of staples, which is somewhat stiff. Moreover, after the operation, the incidence of clinical stenosis at the site of the anastomosis is not rare.

The object of the invention is to provide a surgical stapling instrument for performing an anastomosis, in which said instrument is capable of being removed from the anastomotic site without applying unwanted tissue tension and, in doing so, which reduces the risk of undesirable clinical complications during the healing process.

This object is achieved by a surgical stapling instrument having the features of claim 1. Advantageous versions of the invention follow from the dependent claims.

The surgical stapling instrument according to the invention comprises a frame having a body portion and a handle as well as a staple fastening assembly in the distal region of the instrument. The staple fastening assembly includes a cartridge device, which comprises at least one closed row of staples, and an anvil. The anvil is movable relative to the cartridge device and is adapted to cooperate with the cartridge device for forming the ends of the staples exiting from the cartridge device. A moving device is adapted to move the anvil relative to the cartridge device. A staple driving device is adapted to drive the staples out of the cartridge device towards the anvil. A knife, which has a closed cutting edge, is contained within the cartridge device and is positioned such that there is at least one closed row of staples on the outside of the cutting edge. A knife actuating device is adapted to move the knife towards the anvil. So far, these features are known from the prior art, e.g., from U.S. patent No. 5 205 459.

According to the invention, the line defining the closed row of staples has a plurality of indentations such that the line defining the closed row of staples has a larger total length than a circle tangent to the outer perimeter of the line defining the closed row of staples.

Thus, the line defining the closed row of staples has a larger total length than the circular line defining the array of staples of a prior art surgical stapling instrument in which the cartridge device has about the same size. Consequently, the length of the anastomosis seam is larger than that of an anastomosis performed by means of a conventional stapling instrument. Because of this increased length, the anastomotic site can assume a larger diameter and is more flexible, so that the anvil can be easily moved through the opening created by the knife, and the surgical stapling instrument can be retracted at the end of the operation more easily. Additionally, the resulting larger anastomosis lumen will alleviate the incidence of clinical complications. On the other hand, if it is sufficient that the total length of the line defining the closed row of staples is comparable to that provided by a conventional stapling instrument, the invention allows the use of a smaller instrument, which generally can be easier inserted into the tubular organ and removed therefrom.

Preferably, the line defining the cutting edge of the knife runs essentially in parallel to the line defining the closed row of staples and is of a length greater than a circle tangent to the outer perimeter of the line defining the cutting edge of the knife. In this case, the knife cuts an opening in the tissue which is adapted to the shape of the line defining the closed row of staples, and the size of the anastomotic opening can be easily increased by reshaping the form of the cutting line to a more or less circular shape.

In an advantageous version of the invention, at least in the proximity of the closed row of staples, the circumferential line of the cartridge device has a plurality of indentations. Preferably, the circumferential line of the cartridge device runs essentially in parallel to the line defining the closed row of staples. In the end region of the anvil facing the cartridge device, the circumferential line of the anvil can match the circumferential line of the cartridge device. In this design, the outer shape of the cartridge device and the anvil, in cross-sectional planes, resembles the shape of the line defining the closed row of staples, which generally facilitates application of the surgical stapling instrument.

Preferably, the line defining the closed row of staples is essentially positioned in a plane, which implies that the staple-forming surface of the anvil has a planar configuration as well. This conforms to the general structure of the prior art surgical stapling instruments, and conventional designs for, e.g., the staple driving device can be applied.

In an advantageous version of the invention, the staple fastening assembly is removably mounted in the distal end region of the body portion. This enables, e.g., the staple fastening assembly to be exchanged during a surgical operation or to be designed as a disposable part (whereas the frame including a major part of the mechanical components is sterilizable and re-usable). Moreover, the cartridge device can comprise a removable cartridge containing the staples, such that, e.g., an empty cartridge can be replaced by a fresh one, if required, or the cartridge device can be designed as a re-usable component. Preferably, the anvil is removable as well, which is also essential for certain surgical techniques. These features are generally known from the prior art surgical stapling instruments.

Generally, the term "staple" is used herein in a very general sense. It includes metal staples or clips, but also surgical fasteners made of synthetic material and similar fasteners. Synthetic fasteners usually have a counterpart (retainer member) held at the anvil. In this sense, the term "anvil" also has a broad meaning which includes, in the case of two-part synthetic fasteners, the anvil-like tool where the retainer members are held, and similar devices.

In the following, the invention is described in more detail by means of an embodiment. The drawings show in
- Figure 1: an isometric view of the distal region of an embodiment of the surgical stapling instrument according to the invention, including the staple fastening assembly, the anvil moved away from the cartridge device, and
- Figure 2: a plan view onto the surface of the cartridge device opposing the anvil.

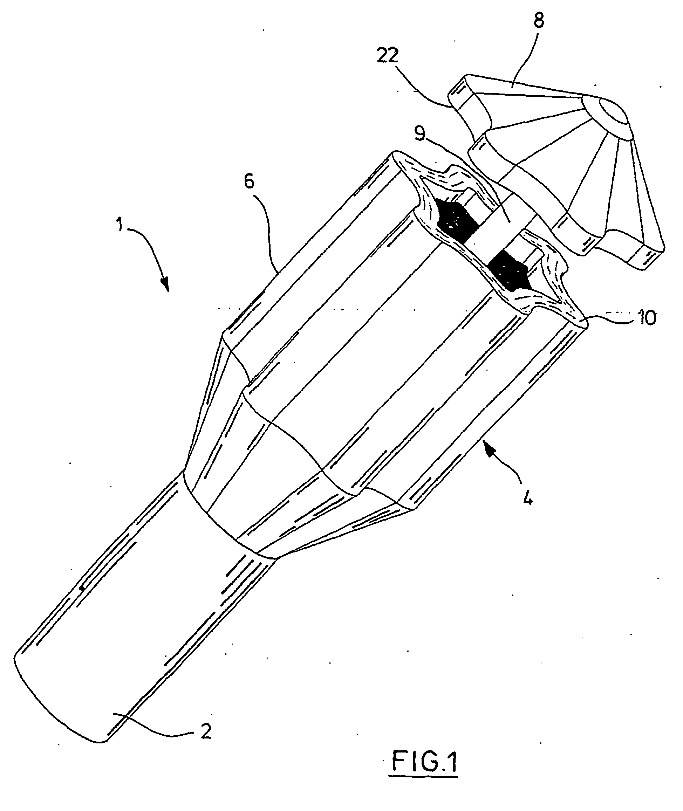

Figure 1 illustrates the distal region of an embodiment of a surgical stapling instrument 1. The instrument 1 includes a frame having a body portion and a handle, and the body portion comprises a shaft 2. A staple fastening assembly 4 is mounted at the distal end of the shaft 2. In the embodiment, the staple fastening assembly 4 can be removed from the shaft 2.

The staple fastening assembly 4 includes a cartridge device 6 which contains a plurality of staples. By means of a staple driving device, which is not described in detail, the staples can be driven out of the cartridge device 6 in a direction which runs essentially parallel to the longitudinal axis of the shaft 2.

An anvil 8 having a conically shaped distal end region is arranged opposite the cartridge device 6. The anvil 8 is supported by a shaft 9 and can be moved by means of a moving device (which is not shown in detail) towards the cartridge device 6 and away from it. In the embodiment, the anvil 8 including the shaft 9 can be separated from the cartridge device 6 when the gap between the cartridge device 6 and the anvil 8 has about its maximum extent.

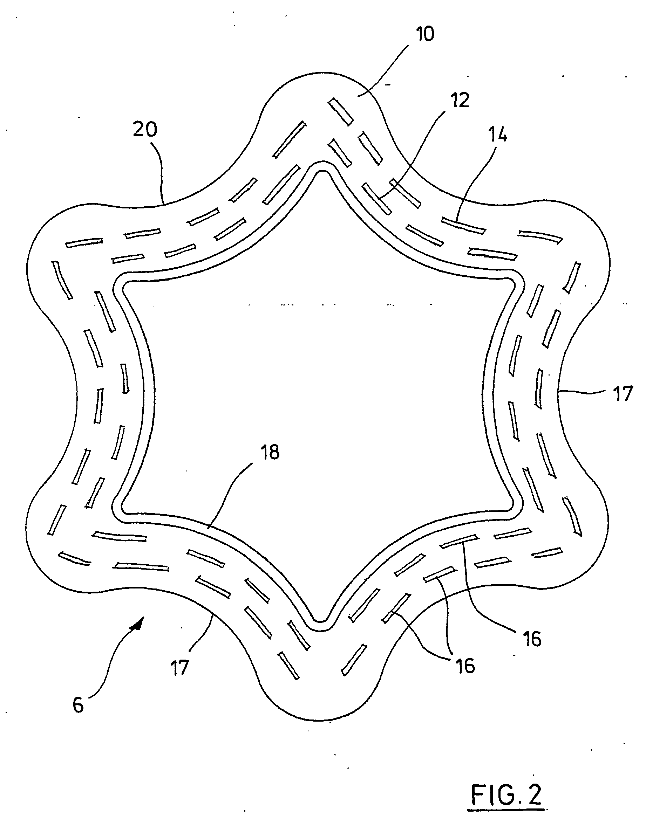

Figure 2 displays a view onto the front plane 10 of the cartridge device 6. The staples are arranged in two closed rows of staples, i.e. in a first closed row 12 and in a second closed row 14. Each individual staple is guided in a slot 16 provided in the cartridge device 6, the pointed ends of the staples facing the anvil 8.

It is evident from Figure 2 that the line defining the first closed row 12 of staples (i.e. the line running, in the plane 10, through the corresponding slots 16 and the gaps between adjacent slots) has a plurality of indentations 17 which increase the overall length of the first closed row 12 of staples. More precisely, the line defining the first closed row 12 has a larger total length than a circle tangent to the outer perimeter of this line. Similarly, the second closed row 14 of staples has a plurality of indentations as well such that this line has a larger total length than a somewhat larger circle, which is tangent to the outer perimeter of the line defining the second closed row 14 of staples. Both closed rows 12 and 14 run essentially in parallel to each other.

Figure 2 shows the cutting edge 18 of a knife which is guided by an inner wall of the cartridge device 6. The cutting edge 18 is closed in order to be able to completely excise a piece of tissue penetrated by the knife. The cutting edge 18 of the knife runs essentially in parallel to the lines defining the closed rows 12 and 14 of staples, which both are arranged outside the cutting edge 18 of the knife.

The outer perimeter 20 or circumferential line of the cartridge device 6 has a similar shape as the closed rows 12 and 14 of staples and the cutting edge 18 of the knife, the corner ridges being somewhat more rounded. In its end region facing the cartridge device 6, the anvil 8 has a similar perimeter 22, see Figure 1.

When the surgical stapling instrument 1 is used, its shaft 2 and the staple fastening assembly 4 are introduced into the hollow organ where the anastomosis is to be performed. Generally, the end region of one part of the organ is placed over the front plane 10 of the cartridge device 6, whereas the end region of the other part is placed over the anvil 8 such that the shaft 9 protrudes and can serve to connect the anvil 8 to the cartridge device 6. By means of the moving device mentioned above, which is operated via actuating elements positioned at or close to the handle of the frame, the anvil 8 is moved relative to the cartridge device 6 until the remaining gap between the anvil 8 and the cartridge device 6 is essentially filled by the tissue of both parts of the hollow organ. In a preferred embodiment of the surgical stapling instrument 1, it is possible to adjust the size of this gap in a well-defined manner. Thereafter, the staple driving device is actuated by means of a trigger close to the handle, in order to drive the staples out of the cartridge device 6 towards the anvil 8. The pointed ends of the staples penetrate the tissue in-between and are bent by the anvil 8 so that the staples are closed. Afterwards, a knife actuating device moves the knife towards the anvil 8 so that the cutting edge 18 of the knife penetrates the tissue. Preferably, the knife actuating device is coupled to the staple driving device so that only one trigger has to be operated in order to perform the functions of stapling and cutting the tissue almost simultaneously. The presence of two closed rows 12, 14 of staples ensures a reliable connection of both parts of the hollow organ.

At the end of the procedure, the surgical stapling instrument 1 can be retracted after the gap between the cartridge device 6 and the anvil 8 is increased in order to release the clamping action. The shape of the closed rows 12, 14 of staples provides a relatively large overall length of the staple lines, so that the tissue at the anastomotic site can yield and the anvil 8 can be easily moved through the opening created by the cutting edge 18 of the knife. The excised tissue portion remains in the surgical stapling instrument 1.

## Claims

1. A surgical stapling instrument comprising:
- a frame having a body portion (2) and a handle,
- a staple fastening assembly (4) in the distal region of said instrument (1), said staple fastening assembly (4) including a cartridge device (6), which comprises at least one closed row (12, 14) of staples, and an anvil (8), which is movable relative to said cartridge device (6) and is adapted to cooperate with said cartridge device (6) for forming the ends of the staples exiting from said cartridge device (6),
- a moving device adapted to move said anvil (8) relative to said cartridge device (6),
- a staple driving device adapted to drive the staples out of said cartridge device (6) towards said anvil (8),
- a knife, which has a closed cutting edge (18), is contained within said cartridge device (6) and is positioned such that there is at least one closed row (12, 14) of staples on the outside of said cutting edge (18), and
- a knife actuating device adapted to move said knife towards said anvil (8),
- **characterized in that** the line defining said closed row (12, 14) of staples has a plurality of indentations such that said line defining said closed row (12, 14) of staples has a larger total length than a circle tangent to the outer perimeter of said line defining said closed row (12, 14) of staples.

2. Stapling instrument according to claim 1, **characterized in that** the line defining said cutting edge (18) of said knife runs essentially in parallel to said line defining said closed row (12, 14) of staples and is of a length greater than a circle tangent to the outer perimeter of said line defining said cutting edge (18) of said knife.

3. Stapling instrument according to claim 1 or 2, **characterized in that**, at least in the proximity of said closed row (12, 14) of staples, the circumferential line (20) of said cartridge device (6) has a plurality of identations.

4. Stapling instrument according to claim 3, **characterized in that** said circumferential line (20) of said cartridge device (6) runs essentially in parallel to said line defining said closed row (12, 14) of staples.

5. Stapling instrument according to claim 3 or 4, **characterized in that**, in the end region of said anvil (8) facing said cartridge device (6), the circumferential line (22) of said anvil (8) matches said circumferential line (20) of said cartridge device (6).

6. Stapling instrument according to one of claims 1 to 5, **characterized in that** said line defining said closed row (12, 14) of staples is essentially positioned in a plane (10).

7. Stapling instrument according to one of claims 1 to 6, **characterized in that** said staple fastening assembly (4) is removably mounted in the distal end region of said body portion (2).

8. Stapling instrument according to one of claims 1 to 7, **characterized in that** said cartridge device (6) comprises a removable cartridge containing said staples.

9. Stapling instrument according to one of claims 1 to 8, **characterized in that** said anvil (8) is removable.

## Patentansprüche

1. Chirurgisches Klammersetzinstrument mit:
- einem Rahmen mit einem Körperbereich (2) und einem Handgriff,
- einer Klammerbefestigungsanordnung (4) im distalen Bereich des Instruments (1), wobei die Klammerbefestigungsanordnung (4) eine Patroneneinrichtung (6), die wenigstens eine geschlossene Reihe (12, 14) von Klammern aufweist, und ein Gegenlager (8) enthält, das relativ zu der Patroneneinrichtung (6) bewegbar ist und dazu eingerichtet ist, zum Umformen der Enden der aus der Patroneneinrichtung (6) austretenden Klammern mit der Patroneneinrichtung (6) zusammenzuwirken,
- einer Bewegungseinrichtung, die dazu eingerichtet ist, das Gegenlager (8) relativ zu der Patroneneinrichtung (6) zu bewegen,
- einer Klammertreibereinrichtung, die dazu eingerichtet ist, die Klammern aus der Patroneneinrichtung (6) auf das Gegenlager (8) zu auszutreiben,
- einem Messer, das eine geschlossene Schneidkante (18) hat, in der Patroneneinrichtung (6) enthalten ist und derart angeordnet ist, dass sich wenigstens eine geschlossene Klammerreihe (12, 14) außerhalb der Schneidkante (18) befindet, und
- einer Messerbetätigungseinrichtung, die dazu eingerichtet ist, das Messer auf das Gegenlager (8) zu zu bewegen,
- **dadurch gekennzeichnet, dass** die die geschlossene Klammerreihe (12, 14) definierende Linie eine Anzahl von Einbuchtungen hat, so dass die die geschlossene Klammerreihe (12, 14) definierende Linie eine größere Gesamtlänge hat als ein Kreis, der tangential zu der äußeren Peripherie der die geschlossene Klammerreihe (12, 14) definierenden Linie verläuft.

2. Klammersetzinstrument nach Anspruch 1, **dadurch gekennzeichnet, dass** die die Schneidkante (18) des Messers definierende Linie im Wesentlichen parallel zu der die geschlossene Klammerreihe (12, 14) definierenden Linie verläuft und eine Länge hat, die größer ist als ein Kreis tangential zu der äußeren Peripherie der die Schneidkante (18) des Messers definierenden Linie.

3. Klammersetzinstrument nach Anspruch 1 oder 2, **dadurch gekennzeichnet, dass** die Umfangslinie (20) der Patroneneinrichtung (6) zumindest in der Nähe der geschlossenen Klammerreihe (12, 14) eine Anzahl von Einbuchtungen hat.

4. Klammersetzinstrument nach Anspruch 3, **dadurch gekennzeichnet, dass** die Umfangslinie (20) der Patroneneinrichtung (6) im Wesentlichen parallel zu der die geschlossene Klammerreihe (12, 14) definierenden Linie verläuft.

5. Klammersetzinstrument nach Anspruch 3 oder 4, **dadurch gekennzeichnet, dass** die Umfangslinie (22) des Gegenlagers (8) in dem der Patroneneinrichtung (6) gegenüberliegenden Endbereich des Gegenlagers (8) zu der Umfangslinie (20) der Patroneneinrichtung (6) passt.

6. Klammersetzinstrument nach einem der Ansprüche 1 bis 5, **dadurch gekennzeichnet, dass** die die geschlossene Klammerreihe (12, 14) definierende Linie sich im Wesentlichen in einer Ebene (10) befindet.

7. Klammersetzinstrument nach einem der Ansprüche 1 bis 6, **dadurch gekennzeichnet, dass** die Klammerbefestigungsanordnung (4) abnehmbar im distalen Endbereich des Körperbereichs (2) montiert ist.

8. Klammersetzinstrument nach einem der Ansprüche 1 bis 7, **dadurch gekennzeichnet, dass** die Patroneneinrichtung (6) eine entfernbare Patrone aufweist, die die Klammern enthält.

9. Klammersetzinstrument nach einem der Ansprüche 1 bis 8, **dadurch gekennzeichnet, dass** das Gegenlager (8) abnehmbar ist.

## Revendications

1. Instrument d'agrafage chirurgical comprenant :
un châssis ayant une partie de corps (2) et une poignée,
un ensemble de fixation d'agrafe (4) dans la région distale dudit instrument (1), ledit ensemble de fixation d'agrafe (4) comprenant un dispositif formant cartouche (6), qui comprend au moins une rangée fermée (12, 14) d'agrafes, et une enclume (8) qui est mobile par rapport audit dispositif formant cartouche (6) et qui est adaptée pour coopérer avec ledit dispositif formant cartouche (6) pour former les extrémités des agrafes sortant dudit dispositif formant cartouche (6),
un dispositif mobile adapté pour déplacer ladite enclume (8) par rapport audit dispositif formant cartouche (6),
un dispositif d'entraînement d'agrafe adapté pour entraîner les agrafes hors dudit dispositif formant cartouche (6) vers ladite enclume (8),
un couteau qui a un bord de coupe fermé (18), est contenu à l'intérieur dudit dispositif formant cartouche (6) et est positionné de sorte que l'on trouve au moins une rangée fermée (12, 14) d'agrafes à l'extérieur dudit bord de coupe (18), et
un dispositif d'actionnement de couteau adapté pour déplacer ledit couteau vers ladite enclume (8),
**caractérisé en ce que** la ligne définissant ladite rangée fermée (12, 14) d'agrafes a une pluralité d'indentations de sorte que ladite ligne définissant de ladite rangée fermée (12, 14) d'agrafes a une longueur totale supérieure à un cercle tangent au périmètre externe de ladite ligne définissant ladite rangée fermée (12, 14) d'agrafes.

2. Instrument d'agrafage selon la revendication 1, **caractérisé en ce que** la ligne définissant ledit bord de coupe (18) dudit couteau s'étend essentiellement parallèlement à ladite ligne définissant ladite rangée fermée (12, 14) d'agrafes et a une longueur supérieure à un cercle tangent au périmètre externe de ladite ligne définissant ledit bord de coupe (18) dudit couteau.

3. Instrument d'agrafage selon la revendication 1 ou 2, **caractérisé en ce qu'**au moins à proximité de ladite rangée fermée (12, 14) d'agrafes, la ligne circonférentielle (20) dudit dispositif formant cartouche (6) a une pluralité d'indentations.

4. Instrument d'agrafage selon la revendication 3, **caractérisé en ce que** ladite ligne circonférentielle (20) dudit dispositif formant cartouche (6) s'étend essentiellement parallèlement à ladite ligne définissant ladite rangée fermée (12, 14) d'agrafes.

5. Instrument d'agrafage selon la revendication 3 ou 4, **caractérisé en ce que**, dans la région d'extrémité de ladite enclume (8) faisant face audit dispositif formant cartouche (6), la ligne circonférentielle (22) de ladite enclume (8) correspond à ladite ligne circonférentielle (20) dudit dispositif formant cartouche (6).

6. Instrument d'agrafage selon l'une quelconque des revendications 1 à 5, **caractérisé en ce que** ladite ligne définissant ladite rangée fermée (12, 14) d'agrafes est essentiellement positionnée dans un plan (10).

7. Instrument d'agrafage selon l'une des revendications 1 à 6, **caractérisé en ce que** ledit ensemble de fixation d'agrafe (4) est monté de manière amovible dans la région d'extrémité distale de ladite partie de corps (2).

8. Instrument d'agrafage selon l'une des revendications 1 à 7, **caractérisé en ce que** ledit dispositif formant cartouche (6) comprend une cartouche amovible contenant lesdites agrafes.

9. Instrument d'agrafage selon l'une des revendications 1 à 8, **caractérisé en ce que** ladite enclume (8) est amovible.
